# EUROPEAN PATENT APPLICATION

(11) **EP 2 327 444 A1**
(43) Date of publication of application: **01.06.2011**
(21) Application number: 10192401.7
(22) Date of filing: 24.11.2010
(51) Int. Cl.: A61M 25/00, B29C 53/56, F16L 3/22

(54) **Package for elongate medical devices**

(30) Priority: 27.11.2009 US 626707
(71) Applicant: Benlan Inc., Oakville, Ontario L6H 5T4 (CA)
(72) Inventor: Enns, Thomas Frederick, Mississauga Ontario L5H 2W8 (CA); Stetieh, Adnan, Mississauga Ontario L4W 3K3 (CA)
(74) Representative: Zinkler, Franz

(57) **Abstract**

A package (20) for elongate medical devices such as guide wires or catheters. The package includes an elongate tube (22) wound into a spiral coil configuration and a first clip (34,36,38,40) overmolded onto the tube such that the first clip surrounds the tube at adjacent sections of the tube to maintain the coil configuration.

## Description

### TECHNICAL FIELD

The present application relates to packaging tubes for elongate medical devices such as catheters and guide wires.

### BACKGROUND DISCUSSION

Elongate medical devices such as catheters and guide wires are typically protected in packaging for transportation and storage prior to use. Such devices are long and thin devices and may be inserted into the lumen of a coiled tube to facilitate transportation and storage.

A tube utilized for packaging such medical devices may be wound into a spiral coil and plastic clips snapped onto the spiral coil, over adjacent sections of the tube. The clips have adjacent openings through which the tube is pushed into place and the mouth of each opening is smaller than the outer diameter of the tube to form an interference fit. After insertion of the medical device, the ends of the tube may be capped and the tube is placed into a sealed, sterile bag.

Such packages have disadvantages, however. Sharp edges or corners of the clips may damage the sealed bag in which the package is placed. Further, the clips fail to maintain the tube in a spiral configuration as a section or sections of the tube may be released from the clip during storage, transportation, or use.

Improvements in packaging for elongate medical devices is desirable.

### SUMMARY

According to one aspect, there is provided a package for elongate medical devices. The package includes an elongate tube wound into a spiral coil configuration and a first clip overmolded onto the tube such that the first clip surrounds the tube at adjacent sections of the tube to maintain the coil configuration.

According to another aspect, there is provided a method of manufacturing the package for elongate medical devices. The method includes locating the tube in a spiral groove in a mold, closing the mold, and overmolding the first clip onto the tube.

Advantageously, a clip or clips are overmolded around the tube at adjacent sections of the tube. The overmolded clips form closed loops around the adjacent sections of the tube, thereby maintaining the tube in a spiral configuration. The overmolded clips may be thermally bonded to the sections of the tube to inhibit telescoping of the tubing from the spiral coil during use. Further, the clips may be low profile and smooth, including chamfered edges to reduce interference with and damage to the bags in which the packages are placed.

Other aspects and features of the present invention will become apparent to those ordinarily skilled in the art upon review of the following description of specific in conjunction with the accompanying figures.

### BRIEF DESCRIPTION OF THE DRAWINGS

Embodiments of the present application will now be described, by way of example only, with reference to the attached Figures, in which:
FIG. 1 is a perspective view of a package for elongate medical devices according to one embodiment of the present invention;
FIG. 2 is a perspective view a portion of the package, including a clip, of FIG. 1, drawn to a larger scale;
FIG. 3 is a front view through of the clip of FIG. 2;
FIG. 4 is a side view of a clip of the package of FIG. 1;
FIG. 5 is a top view of a clip of the package of FIG. 1;
FIG. 6 is a perspective view of a mold including a tube inserted therein for overmolding clips to form the package of FIG. 1;

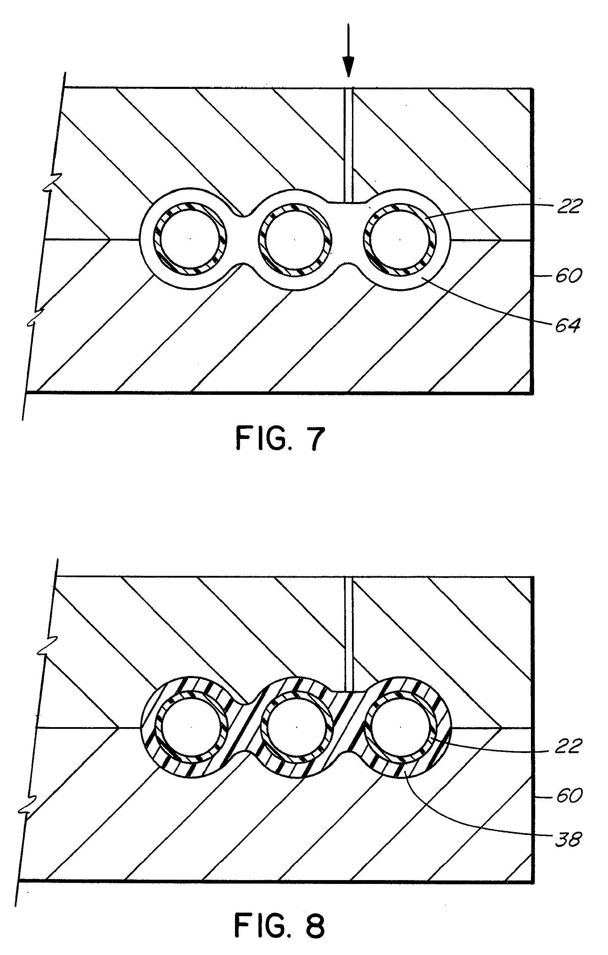

FIG. 7 is a sectional view of the mold of FIG. 6;
FIG. 8 is a sectional view of the mold of FIG. 7, after injection of molding material to form clips.

### DETAILED DESCRIPTION OF EMBODIMENT(S)

It will be appreciated that for simplicity and clarity of illustration, where considered appropriate, reference numerals may be repeated among the figures to indicate corresponding or analogous elements. In addition, numerous specific details are set forth in order to provide a thorough understanding of the embodiments described herein. However, it will be understood by those of ordinary skill in the art that the embodiments described herein may be practiced without these specific details. In other instances, well-known methods, procedures and components have not been described in detail so as not to obscure the embodiments described herein. Also, the present invention is not to be considered as limited to the scope of the embodiments described herein.

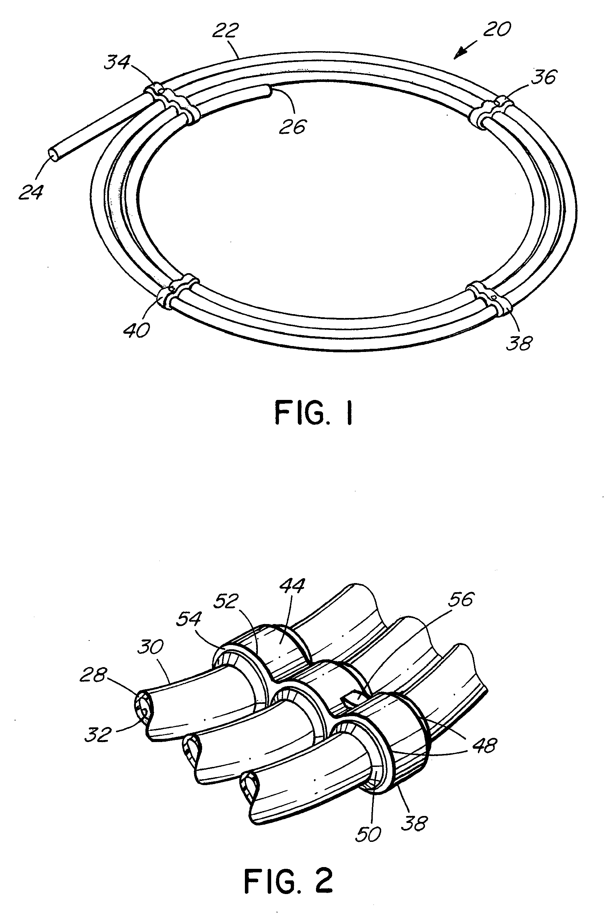

Referring first to FIG. 1, a perspective view of a package for elongate medical devices, such as catheters and guide wires, is shown. The package includes an elongate tube wound into a spiral coil configuration and a first clip overmolded onto the tube such that the first clip surrounds the tube at adjacent sections of the tube to maintain the coil configuration.

Continued reference is made to FIG. 1 to describe the package in detail. The package is indicated generally by the numeral 20 and may be utilized for storage and transportation of the medical devices and for dispensing the medical devices during medical procedures. The package 20 includes an elongate tube 22 with a first end 24 and a second end 26. The tube 22 includes a tube wall 28 that has an outer surface 30, and an inner surface 32 that defines a lumen for receiving an elongate medical device such as a catheter or guide wire. The tube 22 is wound into a spiral coil configuration such that the outer surface 30 of one winding of the coil is adjacent the outer surface 30 of an adjacent winding of the coil. In the present embodiment, the coil includes three windings although other suitable numbers of windings may be utilized.

Four clips 34, 36, 38, 40 are utilized to maintain the tube 22 in the spiral coil configuration. The clips 34, 36, 38, 40 may be made of plastic such as polypropylene and are overmolded onto the tube 22. Each of the clips 34, 36, 38, 40 surrounds adjacent sections of the tube 22 such that each clip forms a closed loop, surrounding the outer surface 30 of the tube 22, at each section at which the clip is located. Thus, there is no opening through which the sections of the tube 22 may be released. Each clip therefore extends in a band around sections of the tube 22.

In the embodiment shown in FIG. 1, four clips 34, 36, 38, 40 are utilized, with the clips 34, 36, 38, 40 spaced generally evenly around the coil configuration. One of the clips 34 is located closer to the ends 24, 26 of the tube 22 than the remaining clips and surrounds four sections of the tube 22, forming a closed loop around each of the four sections of the tube 22. The remaining clips 36, 38, 40 each surround three sections of the tube 22, forming a closed loop around each of the respective three sections.

Each of the clips 34, 36, 38, 40 is thermally bonded to the outer surface 30 at the respective sections of the tube 22 as a result of injection of hot plastic to overmold the clips 34, 36, 38, 40.

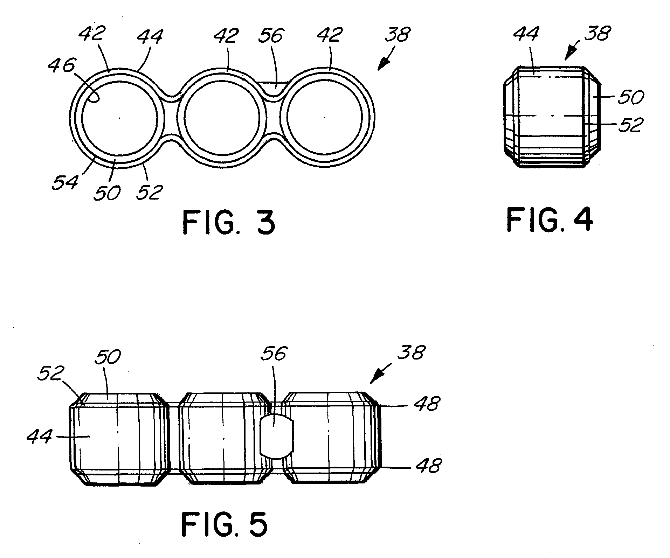

Referring now to FIG. 3 through FIG. 5 in addition to FIG. 1 and FIG. 2, one clip 38 is illustrated. The clip 38 is formed around the sections of the tube 22 to form joined cylinders 42 with an outer cylindrical surface 44 and an inner cylindrical surface 46 that is thermally bonded to the respective section of the tube 22. An additional bridge 56 may be formed between two of the adjacent cylinders 42, where the gate is located during overmolding.

The diameter at the opposing ends 48 of each cylinder of the clip 38, steps down to a reduced diameter portion 50. The edge 52 at the large diameter portion of the step 54 is rounded to provide a smooth surface. The reduced diameter portion 50 is chamfered such that the diameter decreases from the step 54 to the outer surface 30 of the tube 22. The clip 38 is therefore formed with smooth surfaces, reducing the chance of interfering with or damaging the bag in which the package is placed.

Although the clip 34 includes an extra joined cylinder, the remaining features of the clips 34, 36, 40 are similar to those described above with reference to the clip 38 and are not described again herein.

Optionally, a stop clip may be included at or near the end 26, for example. The stop clip may be molded around sections of the tube 22 and may include an additional partial cylinder at the inner winding of the coil. The additional partial cylinder includes a closed end and an opening in the side into which the end 26 may be inserted or removed such that the end 26 abuts or is adjacent the closed end of the clip. The opening in the side is sized to facilitate insertion of the tube end 26 of the tube 22 while maintaining the end 26 in the partial cylinder when inserted into the partial cylinder. The end 26 may be displaced from the partial cylinder by a positive force on the tube 22 to pull the end 26 from the partial cylinder. Thus, the elongate medical device in the tube 22 is inhibited from sliding out of the tube when the end 26 is inserted into the partial cylinder.

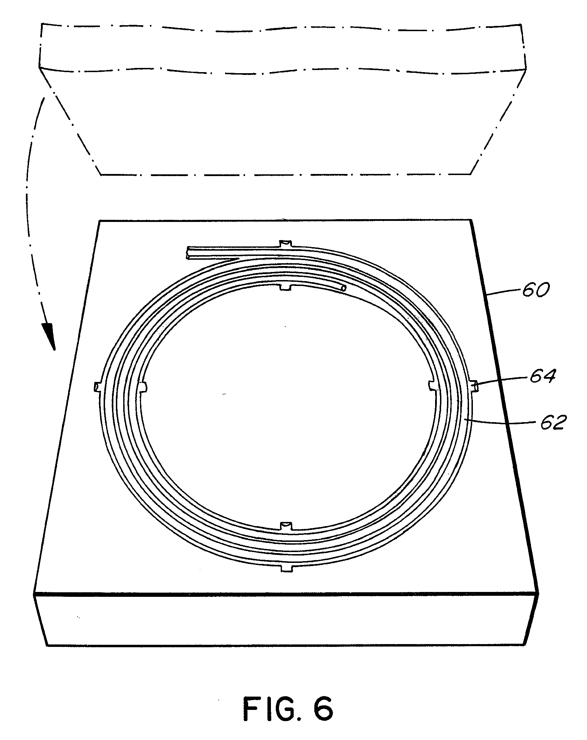

As indicated above, the clips 34, 36, 38, 40 are overmolded onto the tube 22. FIG. 6 shows a perspective view a mold including the tube 22 inserted therein for overmolding the clips 34, 36, 38, 40. One side of the mold 60 includes a spiral groove 62 or slot, that is sized and shaped to receive the tube 22. Four recesses 64 are located for forming the clips 34, 36, 38, 40, with each recess 64 suitably sized and shaped to provide the clips 34, 36, 38, 40. The opposing side of the mold 60 includes complementary slots and recesses for forming the clips 34, 36, 38, 40.

The tube 22 is inserted into one side of the mold utilizing, for example, an automatic feeder which may be controlled by an encoder. When the length of tube is inserted into the mold, the sides of the mold are closed by mating the two sides together and the plastic, such as polypropylene, is injected into the recesses in the mold to form the clips 34, 36, 38, 40 in bands across the coiled tube. After cooling of the injected plastic, the mold is opened and the package is removed.

In other embodiments, different numbers of clips may be utilized. The clips may also take suitable alternative shapes. Further, the number of windings of the tubing may vary, for example, depending on the total length of tubing utilized for packaging.

The above-described embodiments are intended to be examples only. Alterations, modifications and variations can be effected to the particular embodiments by those of skill in the art without departing from the scope of the present application, which is defined by the claims appended hereto.

## Claims

1. A package for elongate medical devices, the package comprising:
an elongate tube wound into a spiral coil configuration; and
a first clip overmolded onto the tube such that the first clip surrounds the tube at adjacent sections of the tube to maintain the coil configuration.

2. The package according to claim 1, wherein the spiral coil configuration comprises at least three windings.

3. The package according to claim 1, wherein the first clip surrounds at least three sections of the tube.

4. The package according to claim 1, comprising further clips overmolded onto the tube such that each of the further clips surrounds adjacent sections of the tube.

5. The package according to claim 1, comprising second, third and fourth clips, each surrounding adjacent sections of the tube.

6. The package according to claim 5, wherein the first, second, third and fourth clips are distributed generally evenly around the coil configuration.

7. The package according to claim 5, wherein the first clip surrounds four sections of the tube and each of the second, third and fourth clips surround three sections of the tube.

8. The package according to claim 1, wherein the first clip is thermally bonded to the sections of the tube.

9. The package according to claim 1, wherein the first clip includes chamfered edges.

10. The package according to claim 1, comprising a stop clip overmolded onto the tube and including a partial cylindrical portion with a closed end into which an end of the tube is releasably insertable.

11. A method of manufacturing the package according to claim 1, the method comprising:
locating the tube in a spiral grove in a mold;
closing the mold; and
overmolding the first clip onto the tube.

12. The method according to claim 11, wherein overmolding comprises overmolding further clips onto the tube such that each of the further clips surrounds adjacent sections of the tube.

13. The method according to claim 11, wherein overmolding comprises overmolding second, third and fourth plastic clips onto the tube.
